# EUROPEAN PATENT APPLICATION

(11) **EP 1 757 278 A1**
(43) Date of publication of application: **28.02.2007**
(21) Application number: 05018269.0
(22) Date of filing: 23.08.2005
(51) Int. Cl.: A61K 9/70, A61K 9/50, A61K 33/00

(54) **Device, system, and method comprising microencapsulated liquid for release of nitric oxide from a polymer**

(71) Applicant: NOLabs AB, 252 21 Helsingborg (SE)
(72) Inventor: Peters, Tor, 8703 Erlenbach (CH)
(74) Representative: Asketorp, Göran

(57) **Abstract**

A device, comprising an NO eluting polymer is provided. Said device is furthermore provided with microencapsulated liquid, such as water or water containing liquid in micro-capsules, which water or water containing liquid after breakage of said micro capsules, initiate elution of NO from said device.

## Description

### Field of the Invention

This invention pertains in general to the field of devices comprising nitric oxide (NO) eluting polymers, involving the use of bound liquid to facilitate and initiate the elution of NO therefrom. More particularly the invention relates to devices manufactured of said NO eluting polymer with bound liquid, a system, comprising said NO eluting polymer and bound liquid, and a process for manufacturing of said device and system.

### Background of the Invention

Nitric oxide (NO) is a highly reactive molecule that is involved in many cell functions. In fact, nitric oxide plays a crucial role in the immune system and is utilized as an effector molecule by macrophages to protect itself against a number of pathogens, such as fungi, viruses, bacteria etc., and general microbial invasion. This improvement of healing is partly caused by NO inhibiting the activation or aggregation of blood platelets, and also by NO causing a reduction of inflammatory processes at the site of an implant.

NO is also known to have an anti-pathogenic, especially an anti-viral, effect, and furthermore NO has an anti-cancerous effect, as it is cytotoxic and cytostatic in therapeutic concentrations, i.e. it has among other effects tumoricidal and bacteriocidal effects. NO has for instance cytotoxic effects on human haematological malignant cells from patients with leukaemia or lymphoma, whereby NO may be used as a chemotherapeutic agent for treating such haematological disorders, even when the cells have become resistant to conventional anti-cancer drugs.

However, due to the short half-life of NO, it has hitherto been very hard to treat viral, bacteria, virus, fungi or yeast infections with NO. This is because NO is actually toxic in high concentrations and has negative effects when applied in too large amounts to the body.

NO is actually also a vasodilator, and too large amounts of NO introduced into the body will cause a complete collapse of the circulatory system. On the other hand, NO has a very short half-life of fractions of a second up to a few seconds, once it is released. Hence, administration limitations due to short half-life and toxicity of NO have been limiting factors in the use of NO in the field of anti-pathogenic and anti-cancerous treatment so far.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible with natural products, after the release of nitrogen oxide.

Other example for NO eluting polymers are given in US-5, 770, 645, wherein polymers derivatized with at least one -NOₓ group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of medical devices to be permanently implanted in the body, such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner to tissues and organs to aid the healing process and to prevent injury to tissues at risk of injury. Electrospun nano-fibers of linear poly(ethylenimine) diazeniumdiolate deliver therapeutic levels of NO to the tissues surrounding a medical device while minimizing the alteration of the properties of the device. A nanofiber coating, because of the small size and large surface area per unit mass of the nanofibers, provides a much larger surface area per unit mass while minimizing changes in other properties of the device.

When using NO eluting polymers, according to above, in medical applications, said polymers need the presence of water to initiate and facilitate the elution of NO. The present inventor has earlier shown that one way to obtain water or moisture in said usage is to place a water bag or sponge in the vicinity of said NO eluting polymer. This water bag or sponge is then broken to set the polymer in contact with water. One may also use the sweat secreted from the skin underneath the medical application or apply water on the medical application after that said medical application has been placed on the area to be treated.

However, even though the idea is genius, the use of a water bag or sponge presents some disadvantages. The water bags need to be delicate, to facilitate breakage by the person using the medical device. This delicacy aggravates transportation and logistic of said medical devices in some extent. Also, the water bag or sponge is somewhat bulky, which impair logistic and use effectiveness. The use of the secreted sweat presents the problem that not all people sweat sufficiently to obtain an adequate elution of NO at the area to be treated. It is a well known fact that some people sweat more than others. Wetting through sweat is also not a time effective way to obtain enough water and/or moisture, since enough secretion of sweat may take some time to obtain.

Hence, an improved device comprising NO eluting polymer, involving the use of bound liquid, such as water or water containing liquid to facilitate and initiate the elution of NO, which liquid is bound in said device in such manner as to eliminate the problems mentioned above in respect of the prior art, would be advantageous.

### Summary of the Invention

Accordingly, the present invention preferably seeks to mitigate, alleviate or eliminate one or more of the above-identified deficiencies in the art and disadvantages singly or in any combination and solves at least the above mentioned problems by providing a device and a system, and a manufacturing method thereof, according to the appended patent claims.

Surprisingly, the present inventor has discovered that it is possible to combine NO eluting polymer and micro encapsulation of a liquid, such as water or a water containing liquid.

Up to this point no one has developed a device or a system comprising an NO eluting polymer and micro encapsulated water or water containing liquid.

In the area of micro encapsulation of liquid, two methods, urea formaldehyde and gelatine capsules, are widely used, but other techniques are also available, which techniques are well known to the skilled artisan. The micro capsules in this technical field may be as small as approximately 8 micrometers and as large as 2 millimeters. They may hold a liquid content of up to approximately 85 %. In this type of micro capsules, the liquid is released by physically rupturing the shell of the micro capsule by pressure, shear forces, or heat.

According to one aspect of the invention, a device is provided comprising an NO eluting polymer and microencapsulated water or water containing liquid, which device may be configured for use as a medical device.

According to another aspect of the present invention a system is provided, which system comprises an NO eluting polymer and microencapsulated water or water containing liquid.

According to another aspect of the invention, a manufacturing process for such a device is provided, wherein the process comprises selecting a plurality of nitric oxide eluting polymeric particles, such as nano fibres, fibres, nano particles, or microspheres, and deploying said nitric oxide eluting particles, and deploying onto said polymeric particles microencapsulated water or water containing liquid.

The present invention has at least the advantage over the prior art that it provides a device and a system that initiates and facilitates elution of NO in a manner that is more prone to withstand transportation, and logistic, and that is pliable to use, hence not bulky.

### Brief Description of the Drawings

These and other aspects, features and advantages of which the invention is capable of will be apparent and elucidated from the following description of embodiments of the present invention, reference being made to the accompanying drawings, in which
Fig, 1 is an illustration of a micro capsule according to the present invention,
Fig. 2 is an illustration of a micro capsule, that has been covered with an NO eluting polymer, according to the invention,
Fig. 3 is an illustration of a mixture of micro capsules and nano-particles or micro spheres according to the present invention,
Fig. 4 is an illustration of a plurality of micro capsules, in for example a film, according to the present invention,
Fig. 5 is an illustration of NO eluting polymer that has been spun onto micro capsules according to the present invention,
Fig. 6 is a planar view of a film of NO eluting polymer that has been combined with a film of micro capsules,
Fig. 7 is a cross-section of a film of NO eluting polymer that has been combined with a film of micro capsules, and
Fig. 8 is an illustration of a combination of films, according to Fig. 6 and 7, that has been applied on a target area.

### Embodiments of the Invention

The following description focuses on embodiments of the present invention applicable to a device, and a system, that for example may be configured for medical applications. However, it will be appreciated that the invention is not limited to this application but may be applied to many other technical fields, wherein elution of NO is sought.

With regard to nitric oxide (nitrogen monoxide, NO), its physiological and pharmacological roles have attracted much attention and thus have been studied. NO is synthesized from arginine as the substrate by nitric oxide synthase (NOS). NOS is classified into a constitutive enzyme, cNOS, which is present even in the normal state of a living body and an inducible enzyme, iNOS, which is produced in a large amount in response to a certain stimulus. It is known that, as compared with the concentration of NO produced by cNOS, the concentration of NO produced by iNOS is 2 to 3 orders higher, and that iNOS produces an extremely large amount of NO.

In the case of the generation of a large amount of NO as in the case of the production by iNOS, it is known that NO reacts with active oxygen to attack exogenous microorganisms and cancer cells, but also to cause inflammation and tissue injury. On the other hand, in the case of the generation of a small amount of NO as in the case of the production by cNOS, it is considered that NO takes charge of various protective actions for a living body through cyclic GMP (cGMP), such as vasodilator action, improvement of the blood circulation, anti-platelet-aggregating action, anti-bacterial action, anti-viral action, anti-inflammatory action, anticancer action, acceleration of the absorption at the digestive tract, renal function regulation, neurotransmitting action, erection (reproduction), learning, appetite, and the like. Heretofore, inhibitors of the enzymatic activity of NOS have been examined for the purpose of preventing inflammation and tissue injury, which are considered to be attributable to NO generated in a large amount in a living body. However, the promotion of the enzymatic activity (or expressed amount) of NOS (in particular, cNOS) has not been examined for the purpose of exhibiting various protective actions for a living body by promoting the enzymatic activity of NOS and producing NO appropriately.

In recent years research has been directed to polymers with the capability of releasing nitrogen oxide when getting in contact with water. Such polymers are for example polyalkyleneimines, such as L-PEI (Linear PolyEthyleneImine) B-PEI (Branched PolyEthyleneImine), PEI-C (PolyEthyleneImine Cellulose), which polymers have the advantage of being biocompatible, after the release of nitrogen oxide. Another advantage is that NO is released without any secondary products that could lead to undesired side effects.

The polymers according to the present invention may be manufactured by electro spinning. Electro spinning is a process by which a suspended polymer is charged. At a characteristic voltage a fine jet of polymer releases from the surface in response to the tensile forces generated by interaction by an applied electric field with the electrical charge carried by the jet. This process produces a bundle of polymer fibres, such as nano-fibres. This jet of polymer fibres may be directed to a surface to be treated.

Furthermore, US 6,382,526, US 6,520,425, and US 6,695,992 disclose processes and apparatuses for the production of such polymeric fibres. These techniques are generally based on gas stream spinning, also known within the fiber forming industry as air spinning, of liquids and/or solutions capable of forming fibers.

Other example for NO eluting polymers are given in US-5,770,645, wherein polymers derivatized with at least one -NOX group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of permanently implanted medical devices, such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner.

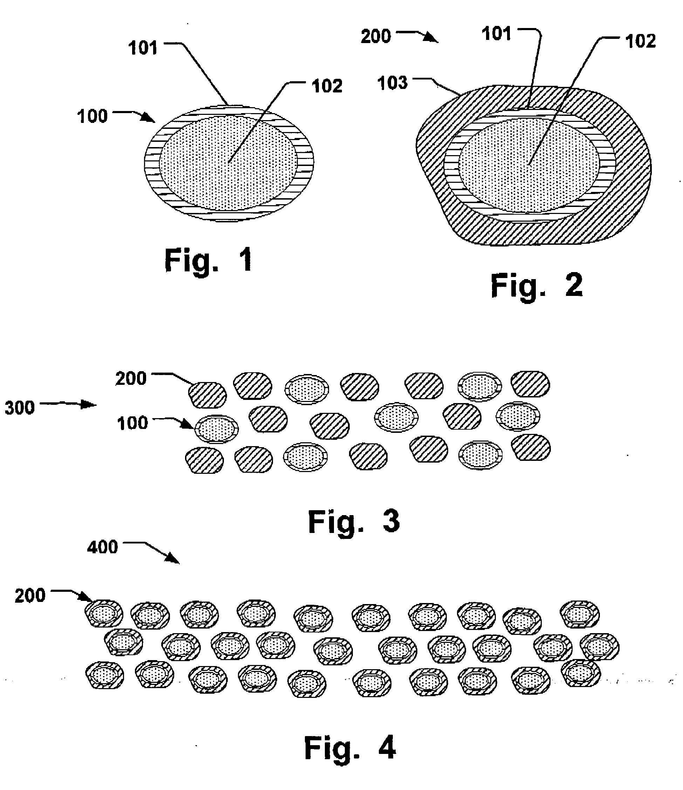

In one embodiment of the present invention an NO eluting polymer, such as polyalkyleneimines, such as L-PEI (Linear PolyEthyleneImine) B-PEI (Branched PolyEthyleneImine), PEI-C (PolyEthyleneImine Cellulose), is provided, and/or combined, with microencapsulated liquid, such as water or water containing liquid, according to Fig. 1. Fig. 1 shows a microcapsule 100, comprising a shell 101 and a microencapsulated liquid 102, such as water or water containing liquid.

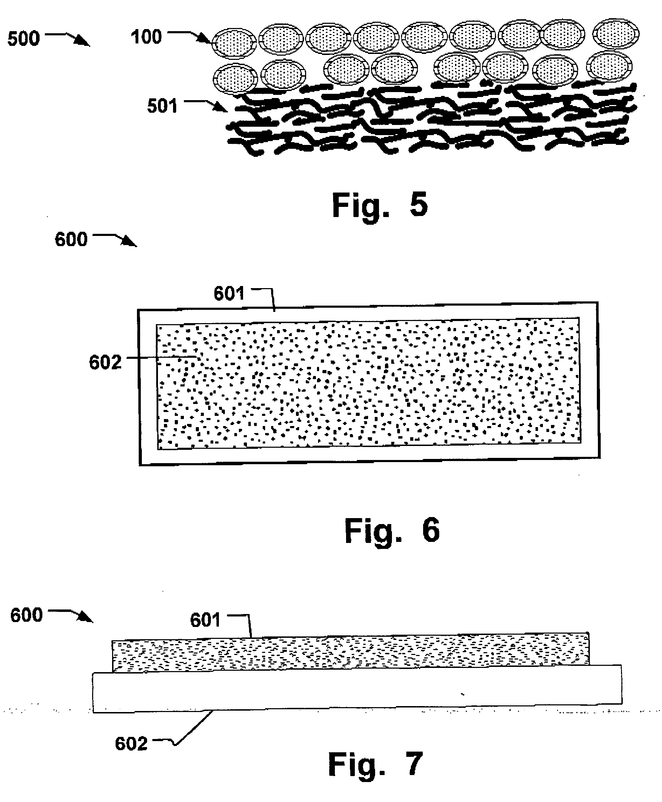

This may for example be done by first manufacture micro capsules, containing water or water containing liquid, in a state of the art manner. These micro capsules may then be formed into a film, tape, sheath, etc. These micro capsules, in form of a film, tape, sheath, etc., are then applied on the NO eluting polymer, according to Fig. 6, which is a planar view of an NO eluting polymer and a film, etc., of micro capsules, and 7, which is a cross section of the configuration in Fig. 6. The application of the micro capsules on the NO eluting polymer may for example be done by gluing, such as pattern gluing, or instead spinning the NO eluting polymer onto said micro capsules. In this way a device or a system, comprising NO eluting polymer and micro encapsulated water or water containing liquid is manufactured. Said device may for example be any device selected from the group; patches, ointments, tapes for cosmetic treatment; tapes, condoms, patches, sheets for treatment of wounds or infections in the oral cavity; patches, socks, condoms for treatment of onychomycosis; patches, socks, tapes, sheets for treatment and/or prevention of neuropathy, such as diabetic neuropathy, diabetic ulcers, vaso-constrictive disorders and macro-angiopathy; condoms, sheets, patches for treatment of rectal disorders, such as fissures, ulcers, haemorrhoids, and levator spasm; devices for target treatment of gastric and gastrointestinal complications, such as gastric ulcer; condom/sheath, tape/coating, fibres, nano-particles, or micro-spheres for wound care; devices for prevention of infection and obtainment of antithrombotic effect; feedstuff or food; and patches etc., for pre-treatment of an area before insertion of a catheter, venflone etc. Said device may for example be any of which are mentioned in the pending European patent applications; 04029796.2, 05006474.0, 05002937.0, 05002935.4, 05002934.7, 05002933.9, 05006495.5, 05006489.8, 05011785.2, and 05011786.0, which pending European patent applications are based on inventions made of the present inventor, and which pending applications hereby are integrated as references in the present application.

Of course, in other embodiments of the present invention, the liquid contained in the micro capsules may be any other liquid, such as alcohol, such as ethanol, etc., or any other polar solvent, with the ability to initiate and produce elution of NO from said NO eluting polymers.

The device or system may then be applied on a target area on which exposure of NO is desired. Such a target area may for example be located on an animal organ, such as the skin, mucous membrane etc, or any other area mentioned and/or described in the pending European patent applications mentioned above.

When the device or system is applied on the target area the device or system is compressed or squeezed. Said compression or squeezing results in breakage of the micro capsules. The NO eluting polymer is thus exposed to said water or water containing liquid, and the elution of NO from the NO eluting polymer is initiated on the target area.

In other embodiments of the present invention the liquid inside the micro capsules is released by heating or shearing the micro capsules until the micro capsules are ruptured.

The elution of NO from said polymer may be used for any conceivable purpose, such as to obtain anti microbial and/or viral effect, vasodilating effect, anti fungal effect, etc.

In another embodiment of the present invention microcapsules, containing water or water containing liquid, are manufactured in a manner according the state of the art. These micro capsules are then covered with an NO eluting polymer, according to above. The covering of the micro capsules is for instance done by spinning the NO eluting polymer onto the micro capsules, containing water or water containing liquid, according to Fig. 2, in which an NO eluting polymer 103 encloses a microcapsule 101. When the combined particle 200 is compressed, or in any other way ruptured, the liquid, such as water or water containing liquid, will get in contact with the NO eluting polymer 103, and thus the elution of NO is initiated. The particles 200 may for example constitute a film, sheath, tape, etc., such as illustrated in Fig. 4.

The spinning may for example be done by air spinning and/or electro spinning. In this way microcapsules covered with NO eluting polymer may be manufactured.

In other embodiments of the invention, the NO eluting polymer may be mixed and manufactured together with other suitable materials, such as polyvinylacetates, polylacticacids, polyesters, polyamides, polyethers, polyurethanes, polycarbonates, cotton, starch, cellulose, polyhydroxyalkanoates, polyesters, polycaprolactone, polyvinylalcohol, polypropylene, polyacrylonitrile, polystyrene, poly(acrylic acid), protein based plastics, gelatine, biogradable polymers, and other soluble plastics. The NO-eluting polymer may be integrated in, spun together with, or spun on top of, any of these materials in all of the embodiments of the present invention. In these embodiments the elution of NO is regulated, such as by decreasing the elution rate, by the admixed materials.

In an embodiment of the invention the NO eluting polymer is in form of nano-particles, or micro spheres. These nano-particles, or micro-spheres, may be formed from the NO-eluting polymers according to the present invention by grinding or in any other way divide the spun polymeric fibres into small parts.

In another embodiment of the device or system according to the present invention, said device or system may be manufactured in the form of a polyurethane, or polyethylene, tape or coating. This polyurethane tape or coating may easily be wrapped around, or applied on, the target area to be treated. At least the side facing the body may be covered with NO-eluting nano-particles, or micro-spheres, or nano-filament of NO-eluting polymer. The covering of NO-eluting nano-particles, or micro-spheres, or nano-filament of NO-eluting polymer is in turn covered with the micro capsules, containing water or water containing liquid. When these particles or filaments get in contact with the water, moisture or water containing liquid inside the micro capsules, after the micro capsules have been compressed or squeezed until the micro capsules break and the water or water containing liquid inside the micro capsules is let out, the NO eluting polymer starts to elute NO.

The increased blood perfusion and vasodilatation, that may obtained from the device or system according to the present invention, may, in another embodiment of the present invention, result in an improved effect when combined with other products, comprising active components. Thus, the synergistic effect from NO and other wound healing, or anti-microbial, anti-inflammatory, or anti-viral, components is within the scope of the present invention.

These fibres, nano-particles, or micro-spheres, may be formed from the NO-eluting polymers comprised in the present invention, for example polyalkyleneimines, such as L-PEI (Linear PolyEthylenelmine), B-PEI (Branched PolyEthyleneImine), and PEI-C (PolyEthyleneImine Cellulose), which polymers have the advantage of being biocompatible, after the release of nitrogen oxide. They may also be encapsulated in any suitable material, such as polyvinylacetates, polylacticacids, starch, cellulose, polyhydroxyalkanoates, polyesters, polycaprolactone, polyvinylalcohol, protein based plastics, gelatine, biogradable polymers, and other soluble plastics.

In a further embodiment of the present invention fibres, nano-particles, or micro-spheres of an NO eluting polymer are mixed with micro capsules, containing water or water containing liquid, according to Fig. 3, wherein fibres, nano-particles, or micro-spheres 200 of an NO eluting polymer are mixed with micro capsules 100. The mixture is then for example applied on a carrier material, such as a tape of polyethylene or any other suitable carrier material. From this tape patches, sheets, or the like, are constructed, which patches, sheets, or the like then are applied on the target area to which elution of NO is desired. It is also possible to produce a film, tape, etc., directly from the mixture of fibres, nano-particles, or micro-spheres 200 and the micro capsules 100.

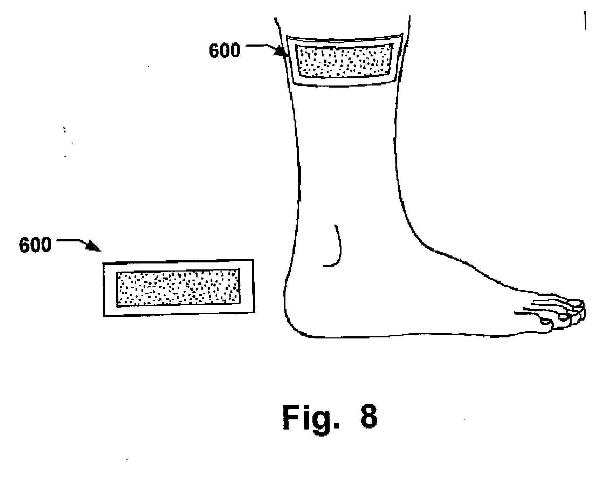

In still another embodiment, according to Fig. 6 and 7, the micro capsules, containing water or water containing liquid, are formed into a film, tape, or sheath 602. Thereafter, a film, tape, or sheath of an NO eluting polymer 601 is glued onto the film, tape, or sheath of micro capsules 602, containing water or water containing liquid. Preferably the film, tape, or sheath of the NO eluting polymer 601 is glued onto the film, tape, or sheath of the micro capsules, containing water or water containing liquid, in patterned way. The obtained pattern includes spaces where there is no glue, in which spaces the water or water containing liquid will be transported to the NO eluting polymer once the micro capsules are broken from compression or squeezing. When the water or water containing liquid gets in contact with the NO eluting polymer the elution of NO starts. Thus, the combination of film, tape, or sheath of micro capsules, containing water or water containing liquid, and NO eluting polymer may be applied on a target area, such as in Fig. 8. Thereafter the combination is compressed or squeezed, which results in that the target area is exposed to NO.

I yet another embodiment the NO eluting polymer is spun directly onto the film, tape, or sheath of micro capsules, containing water or water containing liquid, according to Fig. 5, in which fibres 501 of an NO eluting polymer are spun onto the micro capsules 100. The combination of film, tape, or sheath of micro capsules, containing water or water containing liquid, and spun NO eluting polymer may be applied on a target area. Thereafter the combination is compressed or squeezed, which results in that the target area is exposed to NO.

In still another embodiment of the present invention the device or system is provided with an activation indicator. This activation indicator indicates when the micro capsules are satisfyingly broken, hence when the NO eluting polymer is subjected to enough water or water containing liquid to elute an efficient amount of NO. This activation indicator may for example be obtained by coloring the water or water containing liquid that is trapped inside the micro capsules. When the micro capsules are broken the colored water or water containing liquid escapes the microcapsules and the color gets visualized while efficiently wetting the NO eluting polymer. Another way of obtaining an activation indicator is to choose a manufacture the micro capsules in a material, or choose a wall thickness of said micro particles, that creates a sound when the micro capsules break. It is also possible to admix a scent in the water or water containing liquid, contained in the micro capsules. This results in that the user of the device or system according to the present invention may smell the scent when the water or water containing liquid escapes from the micro capsules after breakage thereof. The released NO may even synergetically augment this scent impression, by itself or by influencing the smell sensing organs, e.g. by vasodilation thereof.

In another embodiment of the present invention the device or system according to the present invention only allows NO-elution in one direction. In this kind of embodiment one side of the device according to the invention is non-permeable to NO. This may be accomplished by applying a material on one side of the device according to the invention that is not permeable to NO. Such materials may be chosen from the group comprising common plastics, such as polyethylene, polyurethane etc. This embodiment is also easy to manufacture as the NO eluting polymer, e.g. L-PEI nano fibres may be electro or gas-jet spun onto the surface of the device according to the invention of e.g. the mentioned plastics, latex, or cotton.

In yet another embodiment of the present invention the NO-eluting device or system is acting as a booster for drug eluting patches, e.g. pharmaceuticals, vitamins, nicotin, nitroglycerin, Non-Steroidal Anti-Inflammatory Drugs (NSAID), such as diclofenac, ibuprofen, aspirin, naproxen, COX-2 inhibitors, choline magnesium trisalicylate, diflunisal, salsalate, fenoprofen, flurbiprofen, ketoprofen, oxaprozin, indomethacin, sulindac, tolmetin, meloxicam, piroxicam, meclofenamate, mefenamic acid, nabumetone, etodalac, ketorolac, celecoxib, valdecoxib, and rofecoxib; steroids, such as cortisone, prednisone, methylprednisolone, prednisolone, vitamin D, estrogen, cholestrol, beclomethasone, flunisolide, fluticasone, triamcinolone, desonide, clobetasol, alclometasole, desoximetasone, betamethasone, halcinonide and dexamethasone; pain reliefs, such as motrin, feldene, naprosyn, lidocaine, and prilocaine; and other substances, such as indinavirsulfate, finasteride, aprepitant, montelukast sodium, alendronate sodium, rofecoxib, rizatriptan benzoate, simvastatin, finasteride, ezetimibe, caspofungin acetate, ertapenem sodium, dorzolamide hydrochloride, timolol maleate, losartan potassium, and hydrochlorotiazide; etc. This embodiment presents a device with the advantage of combining two treatments, of significant value, in one treatment.

Hence, when the device or system according to the present invention is used as a medical application, such device or system may achieve a synergetic effect, when NO is eluted from said device or system. NO has a vasodilatory effect on the region where the device having the combination compound actuates. Vasodilated tissue is more susceptible to certain medications and thus more easily treated by the medical preparations and still NO has in addition to that the anti-inflammatory, anti-bacterial etc. effect. Hence, an unexpected surprisingly effective treatment is provided.

The device or system according to the present invention elutes nitric oxide (NO) from said eluting polymer in a therapeutic dose, such as between 1 to 100 ppm, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.

In the embodiments of the present invention it may be suitable to control or regulate the time span of NO release from the device according to the invention. This may be accomplished by integrating other polymers or materials in said device. These polymers or materials may be chosen from any suitable material or polymer, such as polyvinylacetates, polylacticacids, starch, cellulose, polyhydroxyalkanoates, polyesters, polycaprolactone, polyvinylalcohol, protein based plastics, gelatine, biogradable polymers, and other soluble plastics.

The NO-eluting polymers in the device or system according to the present invention may be combined with silver, such as hydroactivated silver. The integration of silver in the devices according to the present invention gives the healing process an extra boost. Preferably the silver is releasable from the devices in the form of silver ions.

The device or system according to the present invention may be manufactured by, for example electro spinning of L-PEI or other polymers comprising L-PEI or being arranged in combination with L-PEI. L-PEI is the charged at a characteristic voltage, and a fine jet of L-PEI releases as a bundle of L-PEI polymer fibres. This jet of polymer fibres may be directed to a surface to be treated. The surface to be treated may for example be any suitable material in respect of a device according to the present invention. The electro spun fibres of L-PEI then attach on said material and form a coating/layer of L-PEI on the device according to the invention.

It is of course possible to electro spin the other NO-eluting polymers, according to above, on the device according to the invention while still being inside the scope of the present invention.

In one embodiment the NO-eluting polymers according to the present invention are electro spun in such way that pure NO-eluting polymer fibres may be obtained.

It is also within the scope of the present invention to electro spin an NO-eluting polymer together with other suitable polymer/polymers.

Gas stream spinning, or air spinning, of said NO-eluting polymers onto a film of microencapsulated water or water containing liquid or a combination of microencapsulated water or water containing liquid and any suitable NO eluting or non NO eluting polymer is also within the scope of the present invention.

The manufacturing process according to the present invention presents the advantages of large contact surface of the NO-eluting polymer fibres with the area to be covered with NO eluting polymer, effective use of NO-eluting polymer, and a cost effective way of producing the device or system according to the present invention.

Hereinafter, some potential uses of the present invention are described:
A method of treating an animal organ, comprising
   applying a device or system, that comprises a nitric oxide (NO) eluting polymer configured for eluting a therapeutic dosage of nitrogen oxide (NO) when used for said treatment and micro capsules, containing water or water containing liquid, rupturing said micro capsules to set said water or water containing liquid in contact with said NO eluting polymer, and thereby exposing said organ to said nitric oxide when said polymer in use elutes nitrogen oxide (NO) by eluting a therapeutic dose of nitric oxide from said nitric oxide eluting polymer to said treatment site.

The method according to above, wherein said site of said at least one wound is a head, face, neck, shoulder, back, arm, hand, stomach, genital, thigh, leg, or foot of an animal, such as a human, of a body, and wherein said method comprises applying a device, according to above, to said head, face, neck, shoulder, back, arm, hand, stomach, genital, thigh, leg, or foot, for said exposure.

Use of nitric oxide (NO) in a therapeutic dose for therapeutically treating and/or preventing at least one part of an organ.

The invention may be implemented in any suitable form. The elements and components of the embodiments according t'o the invention may be physically, functionally, and logically implemented in any suitable way. Indeed, the functionality may be implemented in a single unit, in a plurality of units, or as part of other functional units.

Although the present invention has been described above with reference to specific embodiments, it is not intended to be limited to the specific form set forth herein. Rather, the invention is limited only by the accompanying claims and, other embodiments than the specific above are equally possible within the scope of these appended claims.

In the claims, the term "comprises/comprising" does not exclude the presence of other elements or steps. Furthermore, although individually listed, a plurality of means, elements or method steps may be implemented. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A device, comprising an NO eluting polymer configured for elution of nitric oxide (NO) therefrom upon moistening of said polymer,
**characterized in that**
said device is provided with a liquid configured for said moistening; wherein
said liquid is microencapsulated in micro capsules,
said micro capsules containing said liquid are arranged to release at least a part of said liquid after breakage of said micro capsules, and
said micro capsules are arranged such that said liquid, when released after said breakage, at least partly moistens said polymer such that elution of NO from said polymer is initiated.

2. The device according to claim 1, wherein said NO eluting polymer is a polyalkyleneimine loaded with NO.

3. The device according to claim 2, wherein said polyalkyleneimine is L-PEI, B-PEI, or PEI-C.

4. The device according to claim 1, wherein said microencapsulated liquid is microencapsulated in formaldehyde and gelatine micro capsules.

5. The device according to claim 1, wherein said NO eluting polymer is spun onto a film comprising said liquid microencapsulated in said microcapsules.

6. The device according to claim 1, wherein said NO eluting polymer is mixed with said micro capsules containing said liquid.

7. The device according to claim 6, wherein said polymer configured for elution of NO is provided in form of fibres, nano-particles and/or micro-spheres.

8. The device according to claim 1, wherein said NO eluting polymer is provided in form of a film, sheath or tape, which is attached onto a film, sheath, or tape comprising said liquid microencapsulated in said microcapsules.

9. The device according to claim 8, wherein said attachment is performed with glue.

10. The device according to claim 9, wherein said glue is applied in a pattern allowing for the liquid inside said micro capsules to get in contact with said NO eluting polymer after breakage of said micro capsules.

11. The device according to claim 1, wherein said device is supplied with an activation indicator.

12. The device according to claim 11, wherein said activation indicator is in form of a color, scent, and/or sound indicator.

13. The device according to claim 1, wherein said liquid is chosen from the group comprising water, water containing liquid, or alcohol, such as ethanol,

14. A system, configured for use in a medical treatment device, **characterized by**
an NO eluting polymer configured for elution of nitric oxide (NO) therefrom upon moistening of said polymer, and
a liquid microencapsulated in micro capsules,
which liquid, after breakage of said micro capsules, initiates elution of NO from said NO eluting polymer.

15. The system according to claim 14, wherein said NO eluting polymer is a polyalkyleneimine loaded with NO.

16. The system according to claim 15, wherein said polyalkyleneimine is L-PEI, B-PEI, or PEI-C.

17. The system according to claim 14, wherein said microencapsulated liquid is microencapsulated in formaldehyde and gelatine micro capsules.

18. The system according to claim 14, wherein said NO eluting polymer is provided in form of fibres, nano-particles and/or micro-spheres.

19. The system according to claim 14, wherein said system is supplied with an activation indicator.

20. The system according to claim 19, wherein said activation indicator is in form of a color, scent, and/or sound indicator.

21. The system according to claim 14, wherein said liquid is chosen from the group comprising water, water containing liquid, or alcohol, such as ethanol.

22. A manufacturing process for a device according to claim 1, comprising:
selecting a plurality of NO eluting polymeric particles, preferably nano fibres, nano particles or micro spheres, and
deploying said NO eluting particles onto a micro capsules containing a micro encapsulated liquid, to form said device,
wherein said deploying comprises electro, air, or gas stream spinning of said particles.

23. A method of activating nitric ocide (NO) elution from an NO eluting polymer configured for elution of nitric oxide (NO) therefrom upon moistening of said polymer, comprising
arranging said NO eluting polymer in the vicinity of micro capsules containing a liquid, and
releasing said liquid by rupturing said micro capsules for moistening said polymer.

24. The method according to claim 23, wherein said rupturing is performed with pressure, shear, or heat.
